# EUROPEAN PATENT APPLICATION

(11) **EP 4 806 472 A2**
(43) Date of publication of application: **16.09.2026**
(21) Application number: 26180368.8
(22) Date of filing: 13.03.2014
(51) Int. Cl.: A61L 31/14

(54) **POLYMER SYSTEM FOR SECURING IMPLANTS IN SYRINGE NEEDLES**

(30) Priority: 14.03.2013 US 201361784502 P; 15.03.2013 US 201361789313 P
(62) Document number(s) of the earlier application(s) in accordance with Art. 76 EPC: 19209979.4 / 3 632 482; 14714136.0 / 2 968 695
(71) Applicant: ALLERGAN, INC., North Chicago, IL 60064 (US)
(72) Inventor: Spada, Lon T., Walnut, CA, 91789 (US); Ghebremeskel, Alazar N., Irvine, CA, 92612 (US); Robinson, Michael R., Irvine, CA, 92606 (US)
(74) Representative: Hoffmann Eitle

(57) **Abstract**

Disclosed herein are methods of delivering implants to a target organ with an implant administration device, where the implant administration device includes a polymer retainer. Methods of making polymer retainers and methods of securing an implant within an implant administration device using a polymer retainer are also disclosed herein.

## Description

### CROSS-REFERENCE TO RELATED APPLICATIONS

This application claims the benefit of U.S. Provisional Application No. 61/784,502 filed on March 14, 2013 and U.S. Provisional Application No. 61/789,313 filed on March 15, 2013. The entire contents of both applications are incorporated herein by reference.

### BACKGROUND

### Field

The disclosure herein related to polymer systems and methods of using polymer systems for securing implants in an implant administration device.

### Description of the Related Art

Implants, such as ocular implants, are often administered to a patient through an implant administration device, such as a syringe needle. Implants, once inserted into the implant administration device, have the potential to fall out of the needle due to stresses from handling of the implant administration device with the implant inside. A hard plug may be used to secure an ocular implant within the needle. However, hard plug systems for securing an implant within a needle can damage the implant or weaken the needle.

### SUMMARY

Disclosed herein are methods of delivering implants to a target organ with an implant administration device, where the implant administration device includes a polymer retainer. Methods of making polymer retainers and methods of securing an implant within an implant administration device using a polymer retainer are also disclosed herein.

According to an embodiment, a method for securing an implant within an implant administration device includes providing an implant administration device comprising an aperture, providing a polymer, providing an implant, inserting the implant within the implant administration device, so that the implant is contained within the implant administration device, and coating or plugging the aperture of the implant administration device with the polymer to form a polymer retainer, thus securing the implant within the implant administration device. In some embodiments, the implant administration device can be a syringe needle, and the syringe needle includes a sharp distal tip. In some embodiments, the polymer is hydroxypropyl methyl cellulose ("HPMC"). According to other embodiments, the sharp distal tip of the syringe is coated with the polymer. In other embodiments, the sharp distal tip is not coated with the polymer. In some embodiments, the syringe needle has a size selected from 22 gauge, 25 gauge, 27 gauge, or 28 gauge. According to some embodiments, the implant is an intraocular implant. According to an embodiment, the polymer retainer increases the amount of actuation force necessary to expel the implant secured within the implant administration device by about 1% to about 25%, compared to actuation force necessary to expel the implant without the polymer retainer present.

According to another embodiment, a method for delivering an implant within an implant administration device to a patient in need thereof includes providing an implant administration device comprising an aperture, providing a polymer comprising HPMC, providing an implant, inserting the implant within the implant administration device, so that the implant is contained within the implant administration device, coating or plugging the aperture of the implant administration device with the polymer to form a polymer retainer, thus securing the implant within the implant administration device, inserting the implant administration device containing the implant and the polymer retainer into an organ of a patient, and administering the implant into the organ of the patient. In some embodiments, the implant administration device can be a syringe needle, and the syringe needle includes a sharp distal tip. In other embodiments, the polymer is provided in a solution or gel form. According to other embodiments, the sharp distal tip of the syringe is coated with the polymer. In other embodiments, the sharp distal tip is not coated with the polymer. In some embodiments, the syringe needle has a size selected from 22 gauge, 25 gauge, 27 gauge, or 28 gauge. According to some embodiments, the implant is an intraocular implant. According to an embodiment, the polymer retainer increases the amount of actuation force necessary to expel the implant secured within the implant administration device by about 1% to about 25%, compared to actuation force necessary to expel the implant without the polymer retainer present.

### BRIEF DESCRIPTION OF THE DRAWINGS

These and other features will now be described with reference to the drawings summarized below. These drawings and the associated description are provided to illustrate one or more embodiments and not to limit the scope of the invention.
Figure 1 illustrates a front view of an example syringe, according to an embodiment.
Figure 2A illustrates an enlarged side view of a syringe needle coated with a polymer retainer, according to an embodiment.
Figure 2B illustrates an enlarged top side view of a syringe needle plugged with a polymer retainer, according to another embodiment.
Figure 3 illustrates a graph comparing the penetration force of 25G needles with and without example polymer retainers.
Figure 4 shows a graph comparing the actuation force of 25G needles with and without example polymer retainers.

### DETAILED DESCRIPTION

An improved method of delivering polymer implants to a target organ, such as an eye, includes inserting an implant into an implant administration device, such as a syringe needle, then applying a polymer retainer to the distal end of the needle loaded with the implant. The polymer retainer can effectively retain the implant within the implant administration device, without significantly increasing the ejection force of the implant. In some embodiments, the polymer retainer can effectively retain the implant within an implant administration device, such as a syringe needle, without reducing the sharpness of the sharp distal tip of the syringe needle. The polymer retainer can advantageously be robust enough to retain the implant within the implant administration device during routine handling of the device, and still allow the implant to be ejected from the device without damaging the implant.

According to some embodiments, the polymer retainer comprises, consists of, or consists essentially of a polymer. In some embodiments, only a single polymer is used. In other embodiments, the polymer retainer comprises, consists essentially of or consists of a mixture of two or more polymers. According to some embodiments, the polymer is a cellulose ether, such as hydroxypropyl methyl cellulose "HPMC". HPMC is commercially available from the Dow Chemical Company under the brand name METHOCEL^{®}. According to other embodiments, the polymer can be hydroxypropyl cellulose, methyl cellulose, hyaluronic acid, polyvidone or povidone, carboxymethyl cellulose, polyethylene oxide, polypropylene oxide, chitosan, agarose, polypeptide, ficoll, or natural and synthetic protein. The polymer retainer may advantageously form viscous solutions, have reasonable adhesion to metals, be water soluble, and biocompatible. The specification sheets, data sheets, and testing data of these polymers are herein incorporated by reference in their entirety.

The polymer may have a suitable molecular weight. In some embodiments, the molecular weight can be 10,000-200,000 daltons.

The polymer may have a suitable aqueous solubility. According to some embodiments, the polymer's solubility in water can be complete. In some embodiments, the aqueous solubility of the polymer can be 40-60 mg/mL.

The polymer may have a suitable glass transition temperature. In some embodiments, the glass transition temperature of the polymer can be in the range of 150°C - 160 °C.

The polymer may have a suitable viscosity. According to some embodiments, the viscosity is in the range of 80000 cps to 120000 cps.

Suitable polymeric materials or compositions for use include those materials which are compatible, that is biocompatible, with the eye so as to cause no substantial interference with the functioning or physiology of the eye. Such materials preferably are at least partially and more preferably substantially completely biodegradable or bioerodible.

According to some embodiments, the polymer retainer can be formulated into a polymer retainer solution or polymer retainer gel before it is applied to an implant administration device. The polymer retainer solution or polymer retainer gel can contain the polymer as well as additional excipients for suitable purposes. In an embodiment, certain excipients may be added to the polymer retainer gel or the polymer retainer solution to modify the viscosity of the solution or gel, so that they can be easily applied to the implant administration device. According to an embodiment, an excipient comprising, consisting essentially of, or consisting of isopropyl alcohol and/or water and/or a buffer can be added to the polymer to form the polymer retainer solution or the polymer retainer gel. When used, one or more excipient can be present in the solution or gel in an amount in the range of 0.2% to 10% by weight of the solution or gel, based on the total weight of the solution or gel.

The implant administration device is a device configured to deliver an implant to an organ of a patient in need thereof. Implants administration devices can include forceps, syringe (equipped with a cannula or needle), trocar, or other suitable device. Suitable devices (apparatus) include those disclosed in U.S. Patent Publication No. 2004/0054374 and U.S. Patent 6,899,717, whose disclosure of such devices are hereby incorporated by reference in their entirety.

According to some embodiments, an implant may be properly sized to fit within the implant administration device. The implant may have a diameter of about 150 µm to about 500 µm.

According to some embodiments, the implant administration device is a syringe needle. A syringe needle may have a sharp, pointed distal end configured to pierce an organ, such as the skin or eye, of a human body. A syringe needle is typically hollow, and can have a needle tip aperture, located near the sharp, pointed distal end of the syringe needle, where substances and bodies, such as an implant, may be sucked in to the needle or expelled out of the needle.

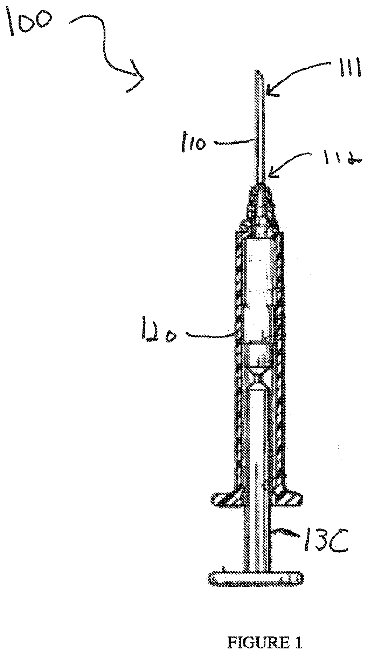

An example syringe needle 100 is illustrated in Figures 1-2B. As shown in Figure 1, syringe needle 100 includes hollow needle 110, syringe body 120, and plunger 130. As shown in Figures 1-2B, hollow needle 110, has a distal end 111, a proximal end 112, and an aperture 113. The distal end 111 has a tip portion 114 which extends from an area just proximal the aperture 115 to a sharp distal tip 116 and includes the aperture 113.

A syringe including an appropriately sized needle, for example, a 22 gauge needle, a 25 gauge needle, a 27 gauge needle, a 28 gauge needle, or a 30 gauge needle, can be effectively used to inject an implant into an organ, such an eye of a human or animal.

An implant, such as an intraocular implant can include a device or element that is configured to be placed in an organ, including those in the ocular region of the eye. Examples include extruded filaments, comprising a biodegradable polymer matrix and an active pharmaceutical ingredient associated with the polymer matrix, and cut to a length suitable for placement in an organ, such as an eye. Implants are generally biocompatible with physiological conditions the body (including, for example, the eye of an animal or human) and do not cause adverse reactions. In some embodiments, implant may be configured for placement in specific areas on an organ, such as the vitreous, anterior chamber, subconjunctival space or sub-tenon space of the eye. Implants, specifically intraocular implants, can be generally biocompatible with physiological conditions of an eye and do not cause adverse side effects. Implants can be biodegradable and may be produced by a suitable process, such as extrusion.

According to some embodiments, an implant may be properly sized to fit within the implant administration device.

The polymer retainer can be applied to an implant administration device, such as a syringe, as a solution, gel, or hot melt either by dipping the needle tip into solution or by direct application of the polymer retainer to the tip of needle. In embodiments where the polymer retainer is applied to the needle tip as a solution or gel, the solution or gel solvent can be removed before the needle containing the polymer retainer is inserted into a patient. In embodiments where the polymer retainer is applied as a hot melt, the melt can be cooled after application to the needle, but before the needle containing the polymer retainer is inserted into a patient.

In embodiments where the polymer retainer is applied to the needle tip by dipping the needle tip into the polymer retainer, the distal end of the needle may be dipped so that the polymer retainer coats the sharp, distal piercing end of the needle tip, up to and including the needle tip aperture. According to some embodiments, the entire needle is not dipped in the polymer retainer. According to some embodiment, the entire length of the needle is not coated with the polymer retainer.

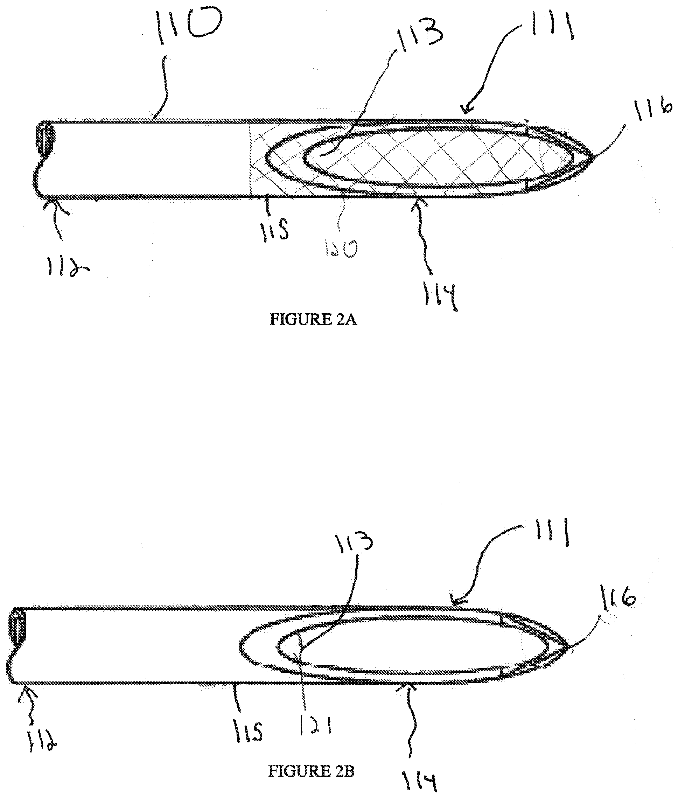

Figures 2A and 2B illustrate example embodiments of implant administration devices, such as needles, using polymer retainers. As illustrated in Figure 2A, the distal end of the needle 111 is coated with polymer retainer 120. As shown by hatching, the polymer retainer 120 coats the tip portion 114 of the distal end 111, and the polymer retainer extends from an area just proximal the aperture 115 to a sharp distal tip 116 and includes the aperture 113. As shown in the embodiment illustrated in Figure 2B, a polymer retainer 113 is a drop or plug in the aperture 113. In the embodiment illustrated in Figure 2B, the sharp distal tip 116 is not coated with the polymer retainer.

According to embodiments where the polymer retainer coats the distal tip of a syringe needle, the coating may have a suitable thickness. In some embodiments, the thickness of the polymer retainer coating, once applied to and dried on the syringe needle can be about 1 µm to about 100 µm.

According to embodiments where the polymer retainer is administered in the aperture of the implant administration device as a plug, a suitable amount of polymer retainer may be used to effectively plug the aperture to contain the implant within the needle, but still beneficially minimize the amount of actuation force necessary to expel the implant from the needle.

In some embodiments, the needle containing a polymer retainer can be used for injections into the ocular region of a patient. The ocular region of a patient may include areas of the patient such as the patient's eyeball and surrounding areas. In some embodiments, the needle containing a polymer retainer can be used for intravitreal, intracameral, or periocular injections.

An improved method of delivering polymer implants to a target organ, such as an eye, includes inserting an implant within an implant administration device, such as a syringe needle, then applying a polymer retainer to the distal end of the needle loaded with the implant.

According to some embodiments, the polymer retainer does not increase the ejection force of the implant of the needle. In some embodiments, the polymer retainer only slightly increases the amount of actuation force necessary to eject an implant contained within a syringe needle. According to some embodiments, the polymer retainer only increases the actuation force necessary to expel an implant contained within a syringe needle by about 1% to about 25%, by about 2% to about 15%, from about 1% to about 10%, from about 1% to about 5%, and the like.

According to some embodiments the polymer retainer does not reduce or only slightly reduces the sharpness of a needle tip. Thus accordingly, in some embodiments, the polymer retainer does not significantly increase the penetration force of the needle necessary to pierce an organ (such as an eye or the skin) of an animal or human, even though the tip portion and sharp distal end of the needle is coated with the polymer retainer. In some embodiments, the polymer retainer only slightly increases the amount of penetration force necessary to pierce an organ (such as an eye or the skin) of an animal or human. According to some embodiments, the polymer retainer only increases the amount of penetration force necessary to pierce an organ by about 1% to about 25%, by about 2% to about 15%, from about 1% to about 10%, from about 1% to about 5%, and the like.

### EXAMPLES

The polymer retainers and methods for applying the polymer retainers to an implant administration device disclosed will now be described by non-limiting example embodiments below.

### EXAMPLE 1

### Manufacturing Method

In an embodiment, example polymer retainers were formulated and applied to the distal end of Hart 25G XTW syringe needles. In a laminar flow hood, 0.25 g HPMC and 9.75 g sterile filtered 70% isopropyl alcohol were added into a Nalgene^{®} plastic sterile bottle and mixed until the HPMC was fully dissolved and a gel was formed.

Two methods were then used to add the polymer retainer to the needles. In a first embodiment, the distal end of the needles were dipped into the gel, allowing the gel fluid to flow into the distal aperture of the needles and into the hollow interior of the needle through capillary action. In a second embodiment, a controlled dispensing system was used to directly deposit the gel onto the distal end of the needle and into the aperture of the needle.

### EXAMPLE 2

### Comparison of Penetration Force

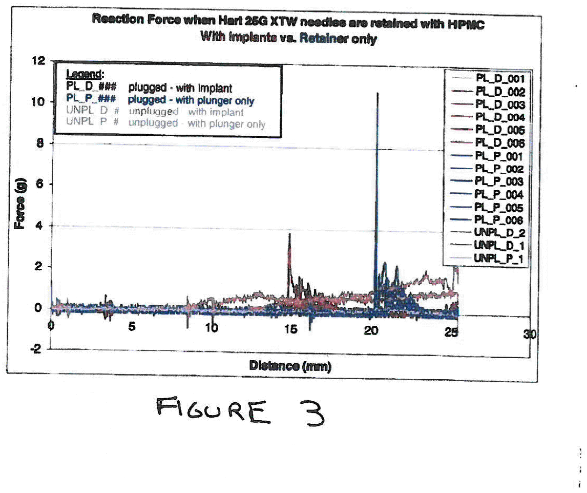

The effect of example embodiment polymer retainers on penetration force was determined by measuring the force required to penetrate a polymer membrane. Penetration force for Hart 25G extra thin wall (XTW) needles was determined using a Texture Analyzer, Stable Micro Systems model TAXT2iHR. A 5 x 0.38 mm implant containing 35% active drug substance and 65% polymer excipients was used as the implant. Penetration force for the polymer retained needles with and without implants is shown in Figure 3. The results show that, surprisingly, both of the methods employed to apply the polymer retainer to the needle tip had no significant effects on the penetration force. A detailed visual inspection of the needle tips showed no significant effect on the needle's sharpness after applying the polymer by either method.

### EXAMPLE 3

### Determination of Ejection Force

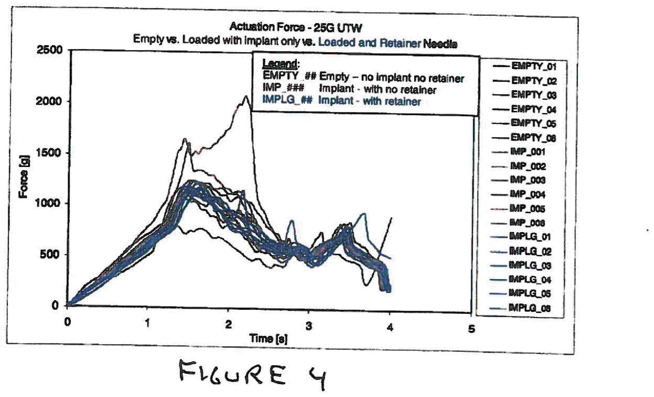

The effect of the polymer retainer on ejection force was determined by measuring the force required to eject a polymer implant from a 25G syringe needle. Ejection force for Hart 25G XTW needles was determined using a Texture Analyzer, Stable Micro Systems model TAXT2iHR. A 5 x 0.38 mm implant containing 35% active drug substance and 65% polymer excipients was used for the tests as the implant. Actuation force for the applicators with and without polymer retained implants are shown in Figure 4. The results show, surprisingly, that the polymer retainer does not require significant additional actuation force.

Although this invention has been disclosed in the context of certain embodiments and examples, it will be understood by those skilled in the art that the present invention extends beyond the specifically disclosed embodiments to other alternative embodiments and/or uses of the invention and obvious modifications and equivalents thereof. In addition while the number of variations of the invention have been shown and described in detail, other modifications, which are within the scope of this invention, will be readily apparent to those of skill in the art based on this disclosure. It is also contemplated that various combinations or subcombinations of the specific features and aspects of the embodiments can be made and still fall within the scope of the invention. Accordingly, it should be understood that various features and aspects of the disclosed embodiments can be combined with, or substituted for, one another in order to perform varying modes of the disclosed invention. Thus, it is intended that the scope of the present invention herein disclosed should not be limited by the particular disclosed embodiments described above, but should be determined only by a fair reading of the claims.

### STATEMENTS:

1. A method for securing an implant within an implant administration device comprising:
   providing an implant administration device comprising an aperture;
   providing a polymer;
   providing an implant;
   inserting the implant within the implant administration device, so that the implant is contained within the implant administration device; and
   coating or plugging the aperture of the implant administration device with the polymer to form a polymer retainer, thus securing the implant within the implant administration device.
2. The method of statement 1, wherein the implant administration device is a syringe needle, the syringe needle comprising a sharp distal tip.
3. The method of statement 1 or 2, wherein the polymer is hydroxypropyl methyl cellulose ("HPMC").
4. The method of statement 2 or 3, wherein the sharp distal tip of the syringe is coated with the polymer.
5. The method of statement 2 or 3, wherein the sharp distal tip is not coated with the polymer.
6. The method of any of statements 2-5, wherein the syringe needle has a size selected from the group consisting of 22 gauge, 25 gauge, 27 gauge or 28 gauge.
7. The method of any of statements 1-6, wherein the implant is an intraocular implant.
8. The method of any of statements 1-7, wherein the polymer retainer increases the actuation force necessary to expel the implant secured within the implant administration device by about 1% to about 25%, compared to actuation force necessary to expel the implant without the polymer retainer present.
9. A method for delivering an implant within an implant administration device to a patient in need thereof comprising:
   providing an implant administration device comprising an aperture;
   providing a polymer comprising HPMC;
   providing an implant;
   inserting the implant within the implant administration device, so that the implant is contained within the implant administration device;
   coating or plugging the aperture of the implant administration device with the polymer to form a polymer retainer, thus securing the implant within the implant administration device;
   inserting the implant administration device containing the implant and the polymer retainer into an organ of a patient; and
   administering the implant into the organ of the patient.
10. The method of statement 9, wherein the implant administration device is a syringe needle, the syringe needle comprising a sharp distal tip.
11. The method of statement 9 or 10, wherein the polymer is provided in a solution or gel form.
12. The method of statement 10 or 11, wherein the sharp distal tip of the syringe is coated with the polymer.
13. The method of statement 10 or 11, wherein the sharp distal tip is not coated with the polymer.
14. The method of any of statements 10-13, wherein the syringe needle has a size selected from the group consisting of 22 gauge, 25 gauge, 27 gauge, or 28 gauge.
15. The method of any of statements 9-14, wherein the implant is an intraocular implant.
16. The method of any of statements 9-15, wherein the polymer retainer increases the actuation force necessary to expel the implant secured within the implant administration device by about 1% to about 25%, compared to actuation force necessary to expel the implant without the polymer retainer present.

## Claims

1. A method for securing an implant within an implant administration device comprising:
providing an implant administration device comprising an aperture;
providing a polymer;
providing an implant;
inserting the implant within the implant administration device, so that the implant is contained within the implant administration device; and
coating or plugging the aperture of the implant administration device with the polymer to form a polymer retainer, thus securing the implant within the implant administration device.

2. The method of Claim 1, wherein the implant administration device is a syringe needle, the syringe needle comprising a sharp distal tip.

3. The method of Claim 1 or 2, wherein the polymer is hydroxypropyl methyl cellulose ("HPMC").

4. The method of Claim 2 or 3, wherein the syringe needle has a size selected from the group consisting of 22 gauge, 25 gauge, 27 gauge or 28 gauge.

5. The method of any of Claims 1-4, wherein the implant is an intraocular implant.

6. The method of any of Claims 1-5, wherein the polymer retainer increases the actuation force necessary to expel the implant secured within the implant administration device by about 1% to about 25%, compared to actuation force necessary to expel the implant without the polymer retainer present.

7. A method for delivering an implant within an implant administration device to a patient in need thereof comprising:
providing an implant administration device comprising an aperture;
providing a polymer comprising HPMC;
providing an implant;
inserting the implant within the implant administration device, so that the implant is contained within the implant administration device;
coating or plugging the aperture of the implant administration device with the polymer to form a polymer retainer, thus securing the implant within the implant administration device;
inserting the implant administration device containing the implant and the polymer retainer into an organ of a patient; and
administering the implant into the organ of the patient.

8. The method of Claim 7, wherein the implant administration device is a syringe needle, the syringe needle comprising a sharp distal tip.

9. The method of Claim 7 or 8, wherein the polymer is provided in a solution or gel form.

10. The method of Claim 2, 3, 8 or 9, wherein the sharp distal tip of the syringe is coated with the polymer.

11. The method of Claim 2, 3, 8 or 9, wherein the sharp distal tip is not coated with the polymer.

12. The method of any of Claims 8-11, wherein the syringe needle has a size selected from the group consisting of 22 gauge, 25 gauge, 27 gauge, or 28 gauge.

13. The method of any of Claims 7-12, wherein the implant is an intraocular implant.

14. The method of any of Claims 7-13, wherein the polymer retainer increases the actuation force necessary to expel the implant secured within the implant administration device by about 1% to about 25%, compared to actuation force necessary to expel the implant without the polymer retainer present.
